# FASCICULE DE BREVET EUROPEEN

(11) **EP 1 638 559 B1**
(45) Date de publication et mention de la délivrance du brevet: **06.01.2010**
(21) Numéro de dépôt: 04767477.5
(22) Date de dépôt: 25.06.2004
(51) Int. Cl.: A61K 31/437, A61P 25/16, A61P 25/28

(54) **UTILISATION DE PYRAZOLOPYRIDINES POUR LE TRAITEMENT DE DEFICITS COGNITIFS**
VERWENDUNG VON PYRAZOLOPYRIDINEN ZUR BEHANDLUNG VON KOGNITIVEN DEFIZITEN
USE OF PYRAZOLOPYRIDINES FOR THE TREATMENT OF COGNITIVE DEFICITS

(30) Priorité: 27.06.2003 FR 0307824
(43) Date de publication de la demande: 29.03.2006
(73) Titulaire: Exonhit Therapeutics S.A., 75013 Paris (FR)
(72) Inventeur: SCHWEIGHOFFER, Fabien, F-94120 FONTENAY SOUS BOIS (FR); DESIRE, Laurent, F-75014 Paris (FR); RESINK, Annelies, b-3001 HEVERLEE (BE); ROUQUETTE, Magali, F-31000 Toulouse (FR)
(74) Mandataire: Becker, Philippe
(86) Numéro de dépôt international: PCT/FR2004/001630
(87) Numéro de publication internationale: WO 2005/000302

(56) Documents cités:
- WO-A-01/78709
- WO-A-01/81345
- WO-A-01/81348
- WO-A-02/098878
- WO-A-03/016563
- WO-A-03/045949
- WO-A-03/076408
- WO-A-2004/045592

## Description

### Domaine de l'invention

La présente invention concerne le domaine de la biologie, de la génétique et de la médecine. Elle concerne notamment de nouvelles compositions et méthodes pour le traitement de pathologies neurodégénératives, et notamment pour améliorer, augmenter ou faciliter la cognition de sujets atteints de pathologies neurodégénératives. L'invention repose plus particulièrement sur l'utilisation de composés de la famille des pyrazolopyridines pour améliorer les facultés cognitives de sujets atteints de maladie neurodégénérative. L'invention est utilisable pour l'amélioration de la condition de sujets atteints de diverses pathologies neurodégénératives, et tout particulièrement de la maladie d'Alzheimer ou de la démence vasculaire.

### Arrière Plan de l'invention

De nombreuses pathologies neurodégénératives ont été décrites comme ayant une composante ou un stade lié au phénomène d'apoptose ou mort cellulaire programmée. On peut citer aussi bien les pathologies neurodégénératives du système nerveux central (par exemple la Sclérose Latérale Amyotrophique - SLA-, la maladie de Parkinson, la maladie d'Alzheimer ou la démence vasculaire), que les maladies dégénératives périphériques, notamment oculaires. Ces pathologies disposent principalement de traitements symptomatiques, notamment de traitements des phénomènes inflammatoires associés, mais peu de traitements des causes réelles de ces désordres, en raison notamment de la complexité des mécanismes et voies métaboliques impliqués, et de la diversité des facteurs causatifs.

La demande internationale de brevet n° WO03/016563, déposée par la demanderesse, décrit de nouvelles cibles moléculaires de la neurotoxicité ainsi que de nouvelles approches thérapeutiques pour le traitement des pathologies neurodégénératives. Ces approches sont basées sur une modulation de l'activité ou de l'expression d'une phosphodiestérase de type 4.

La demande internationale n° PCT/FR04/00366, déposée par la demanderesse, propose de nouvelles approches de traitement des pathologies dégénératives oculaires basées sur une modulation de l'activité ou de l'expression d'une phosphodiestérase de type 4.

Les demandes WO01/78709, WO01/81348, WO01/81345 et WO03/045949 se rapportent à l'utilisation de pyrazolopyridines dans le traitement de certains événements associés aux pathologies neurologiques, tels que la formation d'agrégats peptidiques (WO01/78709), la phosphorylation de la protéine TAU (WO01/81348) ou le blocage de l'enzyme GSK-3 (WO01/81345 et WO03/045949).

La demande WO 02/098878 décrit l'utilisation de trifluorométhylpurines en tant qu'inhibiteurs de la P0E4 pour le traitement des troubles cognitifs.

### Résumé de l'invention

La présente demande concerne maintenant de nouvelles stratégies thérapeutiques de maladies dans lesquelles la perception cognitive est altérée, comme observé notamment dans la maladie d'Alzheimer Ces stratégies sont basées sur une modulation d'une ou plusieurs voies métaboliques identifiées par les inventeurs, qui sont corrélées à l'apparition, au développement et à la progression de l'excitotoxicité et de l'apoptose dans les cellules nerveuses, et sont particulièrement pertinentes dans les maladies neurodégénératives et la fonction cognitive.

La présente demande découle plus particulièrement de la mise en évidence des propriétés avantageuses et remarquables de l'étazolate pour le traitement des déficits cognitifs, notamment ceux induits par la maladie d'Alzheimer. La présente demande propose ainsi de nouvelles stratégies thérapeutiques destinées à traiter ou réduire les troubles cognitifs en particulier chez des patients atteints de maladie neurodégénérative.

La présente invention concerne donc l'utilisation de l'étazolate ou d'un sel ou ester de celui-ci, pour la préparation d'une composition pharmaceutique destinée à améliorer la perception cognitive chez un patient humain.

La présente invention concerne aussi une composition comprenant l'étazolate ou un sel ou ester de celui-ci, pour utilisation pour améliorer la perception cognitive chez un patient humain.

Sans vouloir être lié par un mécanisme d'action, il apparaît que l'action bénéfique inattendue et avantageuse des composés selon l'invention sur les désordres cognitifs puisse s'expliquer par un double impact au niveau du récepteur GABA(A) et de la mitochondrie. En effet, la présente invention décrit l'identification, dans le cerveau de sujets pathologiques, de trois événements moléculaires originaux caractérisés par une altération de l'expression de l'ARNm de la PDE4, de AKAP1 et de GABA(A)RAPL1. Ces événements sont corrélés dans le temps avec le phénomène d'excitotoxicité et/ou de mort neuronale, et démontrent l'existence d'altérations de la signalisation GABA en relation avec des troubles cognitifs. Ainsi notamment, la présente invention révèle l'existence d'altérations d'épissage de l'ARNm codant pour la sous-unité epsilon du récepteur GABA(A) entre des ARNm extraits de cortex préfrontal de patients atteints de la maladie d'Alzheimer d'une part et des ARNm extraits de la même région de cerveau d'individus contrôles ayant le même âge, d'autre part. Cette découverte est particulièrement intéressante puisque cette protéine est impliquée dans la présentation et la désensibilisation du récepteur GABA(A), et que le vieillissement et les processus liés à l'âge sont associés à une augmentation de la durée nécessaire à la désensibilisation de ce récepteur GABA(A). Ces processus, et notamment les déficits cognitifs, pourraient donc être compensés par des composés selon l'invention, présentant une action au niveau de la voie GABA et des mitochondries.

La présente invention apporte donc des éléments nouveaux et essentiels à l'élection du récepteur GABA(A) comme cible thérapeutique pour le traitement de la maladie d'Alzheimer et plus généralement des désordres cognitifs, et permet ainsi de rendre compte des effets biologiques et thérapeutiques observés lors de l'utilisation de composés de la famille des pyrazolopyridines dans le traitement de maladies neurodégénératives, dont la maladie d'Alzheimer et la démence vasculaire, et plus particulièrement pour traiter les désordres cognitifs. Les résultats présentés dans les exemples illustrent notamment l'efficacité de tels composés à améliorer les capacités de mémorisation des animaux dans une situation aversive.

### Description détaillée de l'invention

L'excitotoxicité et l'apoptose sont les deux causes principales de la mort neuronale. Les multiples voies de l'apoptose émanent de la mitochondrie, et un des points cruciaux pour l'apparition de l'apoptose est, par exemple, l'ouverture du pore mitochondrial de transition (MPTP). La surproduction de radicaux libres (ROS), due au dysfonctionnement de la mitochondrie, déséquilibre la régulation de l'apoptose et induit ainsi une augmentation de la vulnérabilité des neurones à l'excitotoxicité.

Ces deux phénomènes, la surproduction des radicaux libres et l'excitotoxicité, sont impliqués dans le mécanisme pathologique entraînant la mort neuronale due à l'âge et les maladies neurodégéneratives telles que la maladie d'Alzheimer, la démence vasculaire, la maladie de Parkinson et la SLA. En effet, il a été démontré que les radicaux libres sont au moins en partie responsables des déficiences des cerveaux âgés. Le stress oxydatif a été impliqué dans la progression de la maladie d'Alzheimer, de la démence vasculaire, de la maladie de Parkinson et de la SLA. Le stress oxydatif est le résultat d'un dérèglement de l'homéostasie entre les pro-oxydants et les anti-oxydants, qui conduit à la génération de radicaux libres toxiques.

Les inventeurs ont établi un répertoire des altérations de l'épissage de l'ARN dans le cerveau d'animaux modèles de SLA âgés de 60 jours, qui a été réalisé par criblage différentiel qualitatif selon la technique DATAS (décrite dans la demande n° WO99/46403). Ce répertoire a été construit à partir d'ARN extraits d'échantillons de cerveau et de moelle épinière, sans isolement préalable des neurones, afin de prendre en compte un maximum d'événements d'épissages alternatifs liés au développement de la pathologie. Le répertoire ainsi produit contient plus de 200 séquences distinctes, impliquant des acteurs clefs du phénomène d'excitotoxicité, tels que les canaux potassiques et le récepteur NMDA. La spécificité des séquences qui constituent ce répertoire est attestée par le fait que la même analyse différentielle qualitative de l'expression génétique réalisée sur des animaux âgés de 90 jours aboutit à un répertoire différent, dont sont absents notamment les différents marqueurs de l'excitotoxicité. L'analyse des modifications d'épissage confirme que les évènements moléculaires sont différents selon le stade de la pathologie.

De manière particulièrement intéressante et inattendue, la réalisation de DATAS sur des ARN d'animaux contrôles et transgéniques âgés de 60 jours a permis d'isoler un fragment d'ADNc dérivé de l'ARNm de la phosphodiestérase 4B, de la protéine AKAP1 ("A Kinase Anchoring Protein") et de la protéine GABA(A)RAPL1 ("GABA(A) Receptor Associated Protein Like 1").

La protéine PDE4B, capable d'hydrolyser l'AMPc, est impliquée dans la régulation de la concentration intracellulaire d'AMPc. La protéine AKAP1 ancre la sous-unité régulatrice de la protéine kinase A (activée par l'AMPc) à la membrane mitochondriale et régule l'activité du pore de transition mitochondrial. Les résultats obtenus montrent une expression plus prononcée de PDE4B dans les tissus nerveux pathologiques, liée à une modification structurale de l'ARN correspondant, notamment à la délétion d'une région dans la partie 3' non-codante. Ce résultat est tout à fait compatible avec la présence de séquences de déstabilisation des ARNm dans la séquence identifiée par DATAS. La délétion de ces séquences de déstabilisation de l'ARNm de la PDE4B, par épissage ou par utilisation de séquences de polyadénylation alternatives, peut aboutir à une stabilisation, donc à une augmentation de l'expression, de la partie codante de cet ARN. Cet événement se produit spécifiquement dans le cerveau des sujets pathologiques et non dans les sujets contrôles.

L'identification d'un fragment dérivé de AKAP1 démontre par ailleurs l'implication de cette protéine dans le développement des processus d'excitotoxicité et de mort neuronale. AKAP1 interagit avec la sous-unité régulatrice de la protéine kinase A et avec le récepteur périphérique aux benzodiazépines (PBR), qui participe à la régulation de l'ouverture du pore mitochondrial de transition, ouverture qui caractérise l'exécution de l'apoptose. Par conséquent l'invention suggère que AKAP1 régule l'intervention du PBR dans les phénomènes de mort cellulaire tels que la mort neuronale.

L'identification d'un fragment dérivé de GABA(A)RAPL1 souligne une dérégulation de la signalisation dépendante du récepteur GABA(A). Cette observation est tout à fait compatible avec l'importance du neurotransmetteur comme inhibiteur de transmission synaptique, notamment par son interaction avec le récepteur GABA(A). Cette inhibition permet de protéger les neurones contre une excitation soutenue qui pourrait conduire à la mort neuronale par excitotoxicité. Nos travaux indiquent donc une altération de ce niveau de régulation, impliqués dans la présentation et la désensibilisation du récepteur GABA(A).

Plus particulièrement, la découverte rapportée dans la présente invention illustre l'existence d'altérations de la signalisation GABA en relation avec des troubles cognitifs. La présente invention révèle aussi l'existence d'altérations d'épissage de l'ARNm codant pour la sous-unité epsilon du récepteur GABA(A) entre des ARNm extraits de cortex préfrontal de patients atteints de la maladie d'Alzheimer d'une part et des ARNm extraits de la même région de cerveau d'individus contrôles ayant le même âge, d'autre part. Aucune anomalie de cette sous-unité n'avait jamais été rapportée jusqu'à présent en pathologie humaine.

La présente invention permet donc de proposer de nouvelles stratégies thérapeutiques des désordres de fonction cognitive, basées sur une modulation de ces voies métaboliques, qui sont corrélées à l'apparition, au développement et à la progression de l'excitotoxicité et de l'apoptose dans les cellules nerveuses, et sont particulièrement pertinentes dans les maladies neurodégénératives.

La présente demande documente les propriétés avantageuses et remarquables de composés de la famille des pyrazolopyridines, dont l'étazolate, pour le traitement des déficits cognitifs, notamment ceux induits par la maladie d'Alzheimer et la démence vasculaire.

Dans le contexte de l'invention, le terme « traitement » désigne le traitement préventif, curatif, palliatif, ainsi que la prise en charge des patients (réduction de la souffrance, amélioration de la durée de vie, ralentissement de la progression de la maladie, amélioration de la survie des neurones, protection des neurones contre l'excitotoxicité ou l'apoptose, etc.), etc. Le traitement peut en outre être réalisé en combinaison avec d'autres agents ou traitements, notamment adressant les événements tardifs de la pathologie, tels que des inhibiteurs de caspases ou autres composés actifs.

L'invention est particulièrement adaptée au traitement des déficits cognitifs chez les sujets, c'est-à-dire à la réduction de ces effets et/ou à l'amélioration de la perception cognitive chez les patients.

L'étazolate répond à la formule (II) suivante :

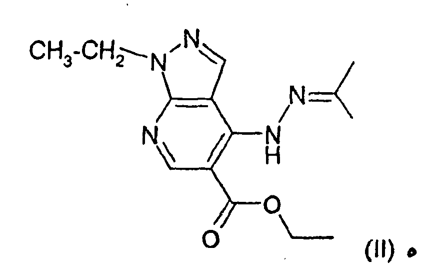

Les composés peuvent être sous forme de sel, ester, racémique, isomère actif, etc. La capacité des composés à protéger les cellules des radicaux libres peut être vérifiée in vitro.

La présente invention propose ainsi, pour la première fois, une intervention thérapeutique liant une modulation des radicaux libres et une modulation du recepteur GABA(A) comme cible thérapeutique pour le traitement des déficits cognitifs associés aux maladies neurodégénératives. Selon des modes de mise en oeuvre particuliers, l'invention peut être utilisée pour traiter les déficits cognitifs en phase précoce de ces maladies. Elle est applicable notamment dans le cas de la maladie d'Alzheimer, de la démence vasculaire, de la chorée de Huntington et de la maladie de Parkinson.

Le composé utilisé dans le cadre de la présente invention peut être formulé et administré de différentes façons. L'administration peut être réalisée par toute méthode connue de l'homme du métier, de préférence par voie orale ou par injection, systémique ou locale. L'injection est typiquement réalisée par voie intra-oculaire, intra-péritonéale, intra-cérébrale, intra-veineuse, intra-artérielle, sous-cutanée ou intra-musculaire. L'administration par voie orale ou systémique est préférée. Les doses administrées peuvent être adaptées par l'homme de l'art. Typiquement, de 0,01 mg à 100 mg / kg environ sont injectés, pour des composés de nature chimique. Des dosages unitaires particuliers sont par exemple de 0,5 à 40 mg par dose administrée. Il est entendu que des injections répétées peuvent être réalisées, éventuellement en combinaison avec d'autres agents actifs ou tout véhicule acceptable sur le plan pharmaceutique (ex., tampons, solutions saline, isotonique, en présence d'agents stabilisants, etc.).

Le véhicule ou excipient acceptable sur le plan pharmaceutique peut être choisi parmi des solutés tampons, solvants, liants, stabilisants, émulsifiants, etc. Des solutés tampons ou diluant sont notamment le phosphate de calcium, sulfate de calcium, lactose, cellulose, kaolin, mannitol, chlorure de sodium, amidon, sucre en poudre et hydroxy propyl méthyl cellulose (HPMC) (pour libération retard). Des liants sont par exemple l'amidon, la gélatine et des solutés de remplissage comme le sucrose, glucose, dextrose, lactose, etc. Des gommes naturelles ou synthétiques peuvent aussi être utilisées, comme notamment l'alginate, la carboxyméthylcellulose, la méthylcellulose, la polyvinyl pyrrolidone, etc. D'autres excipients sont par exemple la cellulose et du stéarate de magnésium. Des agents stabilisants peuvent être incorporés aux formulations, comme par exemple des polysaccharides (acacia, agar, acide alginique, gomme guar et tragacanth, la chitine ou ses dérivés et des éthers de cellulose). Des solvants ou solutés sont par exemple la solution Ringer, l'eau, l'eau distillée, des tampons phosphates, des solutions salines phosphatées, et autres fluides conventionnels.

L'invention est utilisable chez les mammifères, notamment chez l'être humain. Les résultats présentés dans les exemples illustrent l'efficacité de l'étazolate à améliorer la viabilité de neurones placés en conditions d'excitotoxicité, de stress oxydatif ou d'ischémie cérébrale, et à améliorer les capacités de mémorisation des animaux dans une situation aversive.

D'autres aspects et avantages de la présente invention apparaîtront à la lecture des exemples qui suivent.

### LEGENDE DES FIGURES

Figure 1: Effet neuroprotecteur de l'étazolate sur la toxicité induite par NMDA/serine sur les cellules granulaires du cervelet.
Figure 2: Effet neuroprotecteur de l'étazolate sur la toxicité induite par le kainate sur les cellules granulaires du cervelet.
Figure 3: Effet neuroprotecteur de l'étazolate sur la toxicité induite par NMDA/serine sur les neurones corticaux.
Figure 4: Effet neuroprotecteur de l'étazolate sur la toxicité induite par le kainate sur les neurones corticaux.
Figure 5 : Effet neuroprotecteur de l'étazolate sur la toxicité induite par NMDA/sérine sur les cellules de moelle épinière ventrale
Figure 6: Effet neuroprotecteur de l'étazolate sur la toxicité induite par le 6-hydroxydopamine sur les cellules SH-SY5Y
Figure 7 : Effet protecteur de l'étazolate dans le modèle d'infarctus cérébral chez le rat.

### EXEMPLES

### Exemple 1 : Identification de la PDE4, AKAP1 et GABA(A)RAPL1 comme cibles moléculaires de l'excitotoxicité

L'analyse qualitative différentielle a été effectuée à partir d'ARN poly adénylés (poly A+) extraits d'échantillons de cerveaux d'animaux correspondant aux différents stades, sans isolement préalable des neurones afin de prendre en compte un maximum d'évènements d'épissages alternatifs liés au développement de la pathologie.

Les ARN poly A+ sont préparés selon des techniques connues de l'homme de métier. Il peut s'agir en particulier d'un traitement au moyen d'agents chaotropiques tels que le thiocyanate de guanidium suivi d'une extraction des ARN totaux au moyen de solvants (phénol, chloroforme par exemple). De telles méthodes sont bien connues de l'homme du métier (voir Maniatis et al., Chomczynsli et al., Anal. Biochem. 162 (1987) 156), et peuvent être aisément pratiquées en utilisant des kits disponibles dans le commerce. A partir de ces ARN totaux, les ARN poly A+ sont préparés selon des méthodes classiques connues de l'homme de métier et proposées par des kits commerciaux.

Ces ARN poly A+ servent de matrice à des réactions de transcription inverse à l'aide de reverse transcriptase. Avantageusement sont utilisées des reverse transcriptases dépourvues d'activité RNase H qui permettent d'obtenir des premiers brins d'ADN complémentaire de tailles supérieures à ceux obtenus avec des reverse transcriptases classiques. De telles préparations de reverse transcriptases sans activité RNase H sont disponibles commercialement.
Pour chaque point de la cinétique de développement de la pathologie (30 jours, 60 jours et 90 jours) les ARN poly A+ ainsi que les ADNc simple brins sont préparés à partir des animaux transgéniques (T) et des animaux contrôles syngéniques (C).
Conformément à la technique DATAS, pour chaque point de la cinétique sont réalisées des hybridations d'ARNm (C) avec des ADNc (T) et des hybridations réciproques d'ARNm (T) avec des ADNc (C).
Ces hétéroduplex ARNm/ADNc sont ensuite purifiés selon les protocoles de la technique DATAS.
Les séquences d'ARN non appariées avec un ADN complémentaire sont libérées de ces hétéroduplex sous l'action de la RNase H, cette enzyme dégradant les séquences d'ARN appariées. Ces séquences non appariées représentent les différences qualitatives qui existent entre des ARN par ailleurs homologues entre eux. Ces différences qualitatives peuvent être localisées n'importe où sur la séquence des ARN, aussi bien en 5', 3' ou à l'intérieur de la séquence et notamment dans la séquence codante. Selon leur localisation, ces séquences peuvent être non seulement des modifications d'épissage mais également des conséquences de translocations ou de délétions.
Les séquences d'ARN représentant les différences qualitatives sont ensuite clonées selon les techniques connues de l'homme de métier et notamment celles décrites dans le brevet de la technique DATAS.
Ces séquences sont regroupées au sein de banques de cDNA qui constituent des banques qualitatives différentielles. Une de ces banques contient les exons et les introns spécifiques de la situation saine ; les autres banques contiennent les évènements d'épissage caractéristiques des conditions pathologiques.

L'expression différentielle des clones a été vérifiée par hybridation avec des sondes obtenues par reverse-transcription à partir d'ARN messagers extraits des différentes situations étudiées. Les clones hybridant de façon différentielle ont été retenus pour analyse ultérieure. Les séquences identifiées par DATAS correspondent à des introns et/ou à des exons exprimées de façon différentielle par épissage entre les situations pathologiques et la situation saine. Ces évènements d'épissage peuvent être spécifiques d'une étape donnée du développement de la pathologie ou caractéristiques de l'état sain.

La comparaison de ces séquences avec les banques de données permet de classifier les informations obtenues et de proposer une sélection raisonnée des séquences selon leur intérêt diagnostique ou thérapeutique.

La réalisation de DATAS sur des ARN d'animaux contrôles et transgéniques âgés de 60 jours a permis d'isoler un fragment d'ADNc dérivé de l'ARNm de la phosphodiestérase 4B. Ce fragment correspond à un fragment d'exon spécifiquement présent dans les animaux contrôles et donc spécifiquement délété dans les animaux transgéniques pour SOD1 G93A au stade 60 jours. Ce fragment recouvre les nucléotides 377 à 486 référencés à partir du codon stop de la PDE4B de souris (séquence accessible dans GenBank, n°AF208023). Cette séquence comprend 2912 bases, le fragment délété correspondant aux bases 2760 à 2869. Cette région est non codante et est exprimée différentiellement entre les animaux contrôles et les animaux transgéniques, du fait de l'utilisation alternative d'un exon 3' non codant ou du fait de l'utilisation de deux sites de polyadénylation alternatifs.

La réalisation de DATAS sur des ARN d'animaux contrôles et transgéniques âgés de 60 jours a également permis d'isoler un fragment d'ADNc dérivé de l'ARNm de AKAP1. Ce fragment correspond à un fragment d'exon spécifiquement présent dans les animaux contrôles et donc spécifiquement délété dans les animaux transgéniques pour SOD1 G93A au stade 60 jours. Ce fragment est homologue aux nucléotides 1794 à 2322 de la séquence référencée dans GenBank sous le n°NM_009648. Cette région est codante et est exprimée différentiellement entre les animaux contrôles et les animaux transgéniques, du fait d'un épissage alternatif.

La réalisation de DATAS sur des ARN d'animaux contrôles et transgéniques âgés de 60 jours a également permis d'isoler un fragment d'ADNc dérivé de l'ARNm de GABA(A)RAPL1. Ce fragment correspond à un fragment d'exon spécifiquement présent dans les animaux contrôles et donc spécifiquement délété dans les animaux transgéniques pour SOD1 G93A au stade 60 jours. Ce fragment est homologue aux nucléotides 1055 à 1461 de la séquence référencée dans GenBank sous le n°BC024706. Cette région est dérivée de la région 3' non-codante et est exprimée différentiellement entre les animaux contrôles et les animaux transgéniques.

Ces éléments permettent d'élucider et de définir des voies de signalisation importantes, et montrent que la signalisation dépendante du GABA(A)R semble altérée dans le cerveau de patients atteints de la maladie d'Alzheimer. En effet, l'analyse par criblage différentiel qualitatif selon la technique DATAS des ARNm extraits de cortex préfrontal de patients atteints de la maladie d'Alzheimer d'une part et d'ARN extraits de la même région de cerveau d'individus contrôles ayant le même âge, a mis en évidence des altérations d'épissage de l'ARNm codant pour la protéine GABA(A)RAP (GABA(A) Receptor Associated Protein). Cette altération révèle la rétention de 135 bases d'une séquence intronique au niveau de la base 273 de la séquence répertoriée dans GenBank sous le numéro NM_007278.1. Cette rétention modifie la phase ouverte et donc la fonctionnalité de la protéine GABA(A)RAP. Cette protéine étant impliquée dans la présentation et la désensibilisation du récepteur GABA(A), l'analyse DATAS révèle une altération à ce niveau de la régulation des activités synaptiques.
La signalisation GABA représente l'un des plus puissants mécanismes de régulation négative de l'activité synaptique. Lors de la stimulation de ce récepteur GABA(A) par le neuromédiateur GABA, ce récepteur qui est un canal ionique permet l'entrée d'ions chlore qui participent à la repolarisation des neurones. Le récepteur GABA(A) présente une structure pentamérique formée de l'association de 2 sous-unités alpha, deux sous-unités beta et une sous-unité accessoire, delta, epsilon ou gamma principalement.
Les agonistes du récepteur GABA(A) sont anxiolytiques mais amnésiants.
Les antagonistes du récepteur GABA(A) sont anxiogènes, proconvulsants et promnésiants.
De plus, il est connu que, dans le cerveau de patients atteints de la maladie d'Alzheimer, l'une des sous-unités du récepteur GABA(A), la sous-unité beta3, est sous-exprimée.

La sous-unité epsilon, présente dans l'hippocampe et qui est une des premières structures cérébrales à être altérée dans le développement de la maladie d'Alzheimer, confère des propriétés pharmacologiques originales au récepteur GABA(A). En effet, la liaison, via les sous-unités béta, aux récepteurs GABA(A) qui contiennent une sous-unité epsilon, d'agents pharmacologiques comme les pyrazolopyridines, tels le tracazolate et l'étazolate, accélère la désensibilisation du récepteur GABA(A) après interaction avec le neurotransmetteur GABA. Cet effet est particulièrement intéressant puisque le vieillissement est associé à une augmentation de la durée nécessaire à la désensibilisation du récepteur GABA(A).

Une altération de la sous-unité epsilon, comme celle décrite dans la présente invention, associée à l'allongement de la période nécessaire à la désensibilisation des récepteurs GABA(A) dans les processus liés à l'âge comme la maladie d'Alzheimer, peut donc être compensée par le traitement des patients avec des agents pharmacologiques, comme les pyrazolopyridines, tels le tracazolate et l'étazolate. Ce dernier composé présente également l'avantage d'être un inhibiteur de PDE4 dont l'invention montre l'implication dans les phénomènes d'excitotoxicité.

La possibilité d'affecter cette voie de signalisation peut conduire à des traitements particulièrement efficaces des pathologies neurodégénératives, notamment des maladies dégénératives associées à une altération des fonctions cognitives comme la maladie d'Alzheimer et la démence vasculaire.

### Exemple 2 : Inhibition de l'excitotoxicité

Dans cet exemple, des neurones granulaires du cervelet, des neurones corticaux ainsi que des cellules de moelle épinière ventrale de rat ont été mis en culture selon les techniques comme décrit ci-dessous.

### Culture primaire des cellules granulaires de cervelet :

Les rats Wistar âgés de sept jours sont décapités et leurs cervelets sont disséqués. Après avoir enlevé les méninges, le tissu est coupé en petits morceaux et trypsinisé pendant 15 minutes à 37°C. Les cellules sont dissociées par trituration et mises en cultures à une densité 300.000 cellules par cm² dans du milieu basal Eagle supplémenté avec 10% du sérum de veau foetal et 2 mM glutamine. Le lendemain 10 µM ARA-C, un anti-mitotique, est ajouté pour empêcher la prolifération des cellules gliales. Les cellules sont traitées 9 jours après la mise en culture avec le composé inhibiteur étazolate, avant l'addition des toxiques, 50 µM kainate ou 100 µM N-methyl-D-aspartate en présence de 10 µM D-sérine. Le 8-bromo-cAMP est ajouté juste avant les toxiques. Tous les traitements sont effectués au minimum en double et dans au moins deux cultures différentes. Après une incubation de six heures la toxicité est mesurée par un test MTT. Les résultats, normalisés à la moyenne du non-traité, sont statistiquement analysés par le test de Wilcoxon. La valeur significative est déterminée à p inférieur ou égal à 0.05.

### Cultures primaires des cellules corticales :

Des embryons de rat Wistar, âgés de 16 jours, sont prélevés et les cortex sont disséqués. Après la trypsination à 37°C pendant 25 minutes, les cellules sont dissociées par trituration. Les cellules sont ensemencées dans du milieu essentiel minimum, supplémenté avec 10% de sérum de cheval et 10% de sérum de veau foetal et 2 mM glutamine, à une densité de 300.000 cellules par cm². Après 4 jours en culture la moitié du milieu est changée avec du milieu essentiel minimum supplémenté avec 5% de sérum de cheval et 2 mM glutamine. Le même jour, 10 µM de 5-fluoro-2-deoxyuridine, un anti-mitotique, est ajouté. Après sept et onze jours de culture, la moitié du milieu est changée par du milieu conditionné. Le milieu conditionné est composé de MEM contenant 5 % de sérum de cheval et 2 mM glutamine ; ce milieu est passé sur un tapis d'astrocytes corticales pendant une nuit avant son utilisation. A jour 14, les cellules sont traitées avec le composé inhibiteur étazolate, avant l'addition des toxiques, 50 µM kainate ou 20 µM N-methyl-D-aspartate en présence de 10 µM D-sérine. Tous les traitements sont effectués au minimum en double et dans au moins deux cultures différentes. Après une incubation de six heures la toxicité est mesurée par un test MTT. Les résultats, normalisés à la moyenne du non-traité, sont statistiquement analysés par le test de Wilcoxon. La valeur significative est déterminée à p inférieur ou égal à 0.05.

### Cultures primaires de cellules de moelle épinière ventrale :

Les cellules sont isolées à partir d'embryons de rat Wistar âgés de 14 jours. A leur arrivée, les rates gestantes sont sacrifiées par du dioxyde de carbone. Le chapelet d'embryons est prélevé et mis dans une boîte contenant du PBS.
La moelle épinière de chaque embryon est disséquée et la corde ventrale est séparée des cordes dorsales. Les cordes ventrales sont ensuite trypsinisées à 37°C pendant 20 min. L'effet de la trypsine est arrêté par l'addition d'un milieu composé de milieu Leibovitz 15, 20% de sérum de cheval, supplément N2 (1X), 20% de glucose (3.2mg/ml), 7.5% de bicarbonate (1.8mg/ml) et de L-glutamine (2mM). Les cellules sont dissociées par trituration. Les amas tissulaires sont enlevés et les cellules dissociées sont ensuite quantifiées par coloration au bleu de trypan. Les cellules ensemencées à une densité de 250 000 cellules/cm² dans un milieu composé de milieu neurobasal, de sérum de cheval (2%), de supplément B27 (1X), et de glutamine (2mM). Après 3 jours de culture *in vitro,* un agent anti-mitotique, l'ARA-C (5µM), est ajouté aux cellules afin d'inhiber la production de cellules gliales. Les cellules sont mises en culture à 37°C dans un incubateur humidifié (5% CO2) pour 9 jours. Après 9 jours de culture, les cellules sont traitées avec le composé inhibiteur : l'étazolate, avant l'ajout de 25µM de N-methyl-D-aspartate (NMDA) en présence de 10µM D-sérine. Tous les traitements sont effectués au minimum en double et dans au moins deux cultures différentes. Après 3 heures d'incubation avec NMDA/D-serine comme toxique la toxicité est révélée par test MTT.
Les résultats sont normalisés à la moyenne des contrôles non traités et analysés statistiquement par un test de Wilcoxon avec p inférieure à 0.05.

### Test MTT :

La toxicité est mesurée en utilisant le test MTT. Après l'incubation avec les composés, du MTT est ajouté à une concentration finale de 0.5 mg/ml par puits. Les plaques sont ensuite incubées pendant 30 minutes à 37 °C dans le noir. Le milieu est aspiré et les cristaux sont remis en suspension dans 500 µl de DMSO (diméthylsulfoxyde). L'absorbance à 550 nm est lue et le pourcentage de viabilité est calculé.

### Résultats :

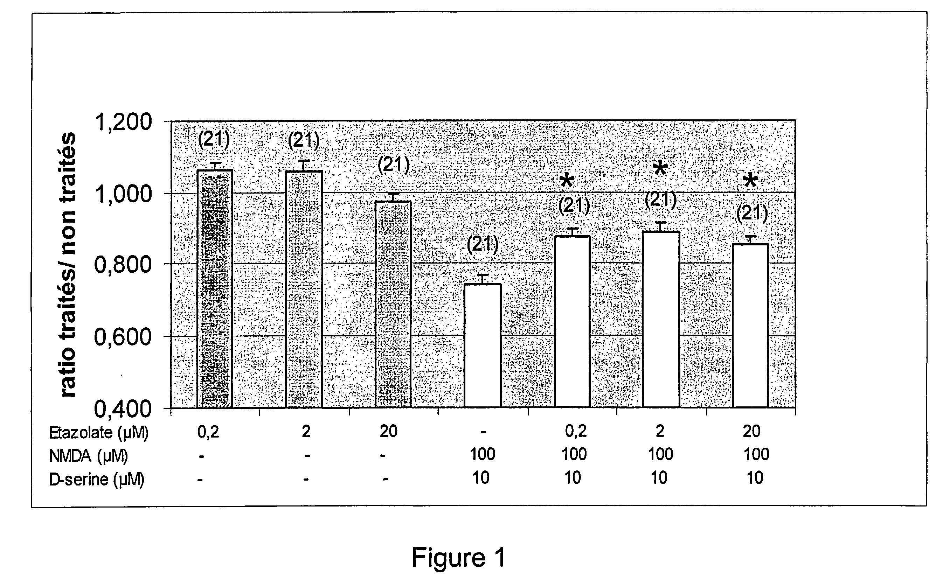

Les résultats obtenus sont présentés sur les figures 1-5. Ces résultats illustrent l'effet protecteur des composés de l'invention sur la survie neuronale. Lors du co-traitement des neurones par un inhibiteur de l'invention, un effet protecteur dose-dépendant est observé dans les modes d'induction de l'excitotoxicité (NMDA/Serine et/ou kalnate).

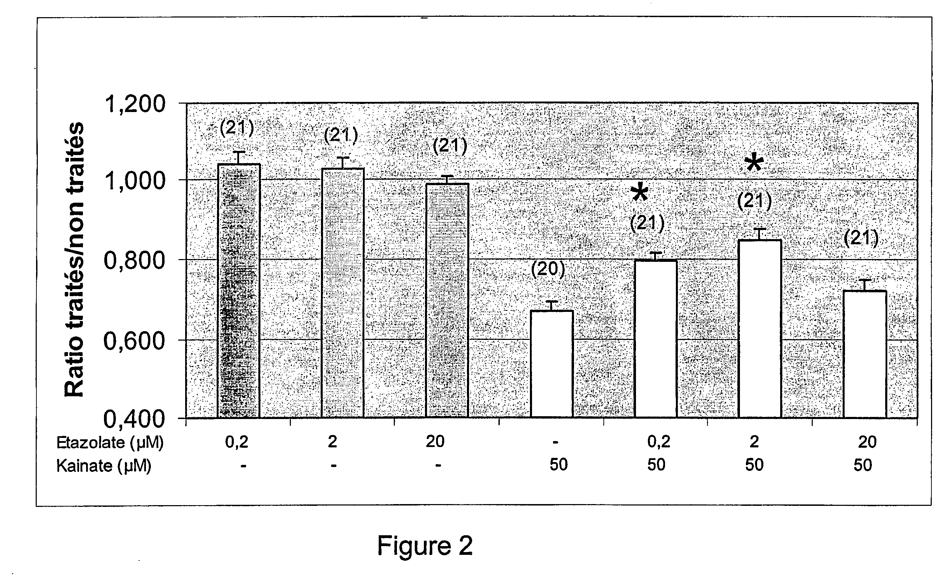

Les figures 1 et 2 présentent des résultats obtenus à l'aide de l'étazolate sur les cellules granulaires du cervelet. Les résultats présentés montrent que l'étazolate permet d'atteindre sur ces cellules un effet protecteur de 40% dans le cas du traitement NMDA/Ser, et de 50% dans le cas de la toxicité induite par le kainate.

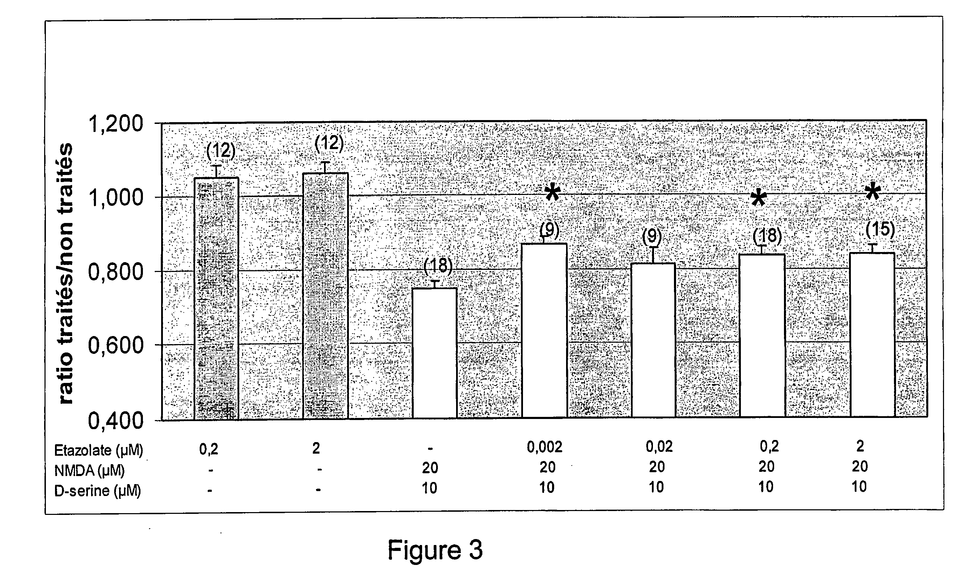

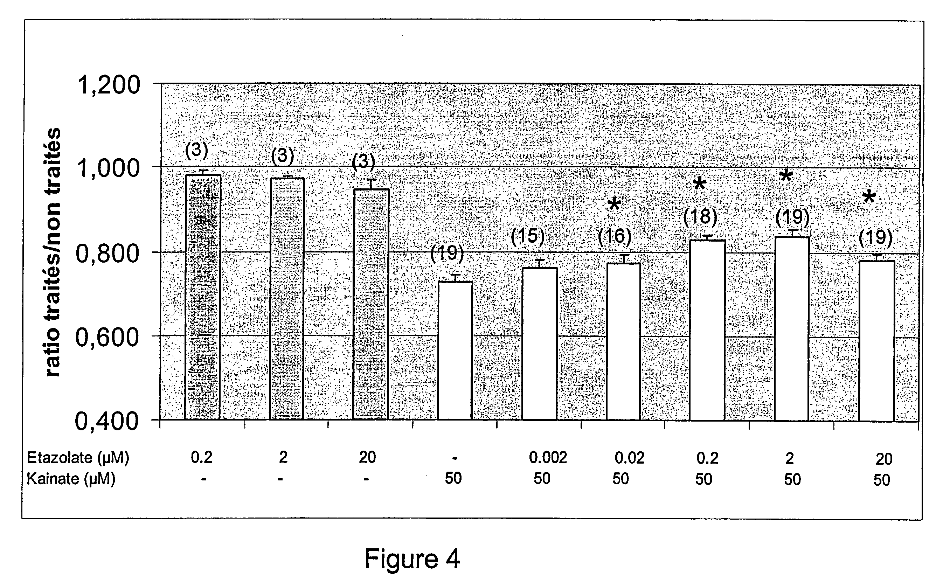

Les figures 3 et 4 présentent des résultats obtenus à l'aide de l'étazolate sur les neurones corticaux. Les résultats présentés montrent que l'étazolate permet d'atteindre sur ces cellules un effet protecteur de 47% dans le cas du traitement NMDA/Ser, et de 40% dans le cas de la toxicité induite par le kainate.

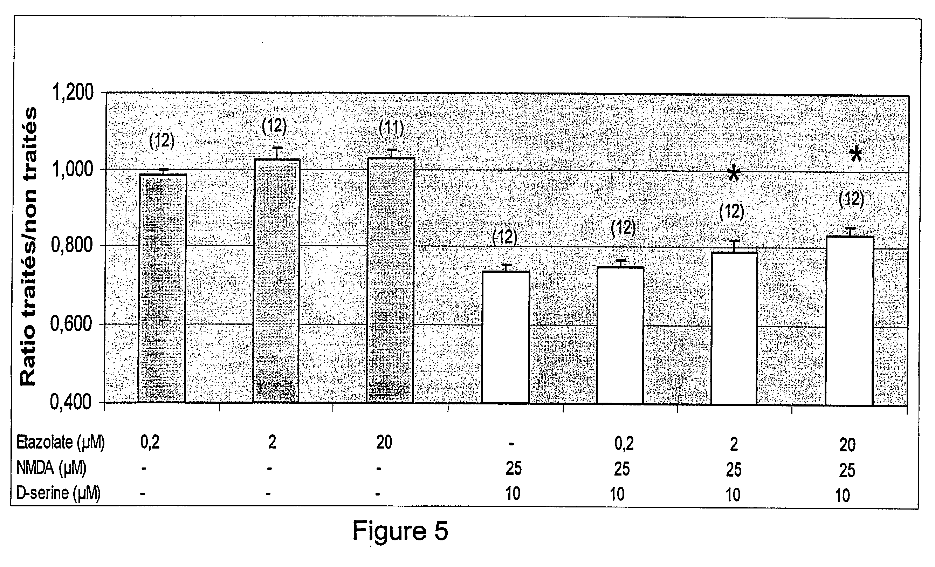

La figure 5 présente les résultats obtenus avec l'étazolate sur les cellules de moelle épinière ventrale. Ces résultats montrent que l'étazolate permet d'atteindre sur ces cellules un effet protecteur de 36% dans le cas du traitement NMDA/Ser.

La présente invention documente donc non seulement l'implication de la PDE4B et des récepteurs GABA(A) dans les mécanismes d'excitotoxicité, mais également la capacité d'inhibiteurs à préserver la viabilité neuronale lors de stress liés à l'excitotoxicité.

### Exemple 3 : Inhibition du stress oxydatif

Dans cet exemple, des cellules de la lignée SH-SY5Y ont été mises en culture selon les techniques connues de l'homme de métier. Ces cellules, dérivées d'un neuroblaste humain, possèdent les propriétés qui caractérisent un précurseur neuronal à un stade précoce du développement.
Le toxique employé est la 6-hydroxydopamine (6-OHDA) qui induit un stress oxydatif. La toxicité est mesurée par un test MTT.

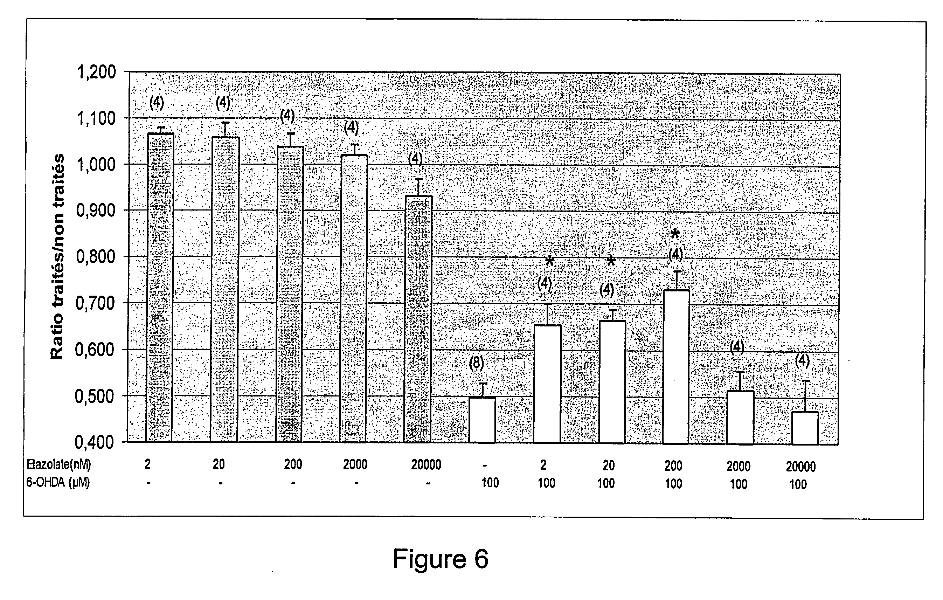

La figure 6 présente les résultats obtenus avec l'étazolate sur les cellules SH-SY5Y. Ces résultats montrent que l'étazolate permet d'atteindre sur ces cellules un effet protecteur de 40% dans le cas du traitement 6-OHDA.

L'étazolate est donc un protecteur potentiel, in vitro, de la mort cellulaire induite par les ROS.
Le potentiel neuroprotecteur d'étazolate est donc renforcé par les résultats obtenus dans l'exemple 2 et 3.

### Exemple 4 : Etude de l'ischémie chez le rat

L'effet protecteur in vivo de l'étazolate a été évalué dans un modèle d'infarctus cérébral chez le rat. Au cours de cette étude, un infarctus cérébral a été provoqué par une occlusion intracavitaire de la carotide interne et des artères moyennes du cerveau. Un groupe de huit rats a été traité avec l'étazolate (10mg/kg, p.o.) avant et plusieurs fois après l'occlusion. Un groupe de huit rats a été traité avec le composé de référence, L-NAME (1 mg/kg, i.p.) avant et plusieurs fois après l'occlusion. Un groupe de huit rats n'a été traité qu'avec le véhicule. Les effets ont été évalués par des observations cliniques, des tests fonctionnels neurologiques et par la détermination de la taille de l'infarctus à la fin de l'étude.

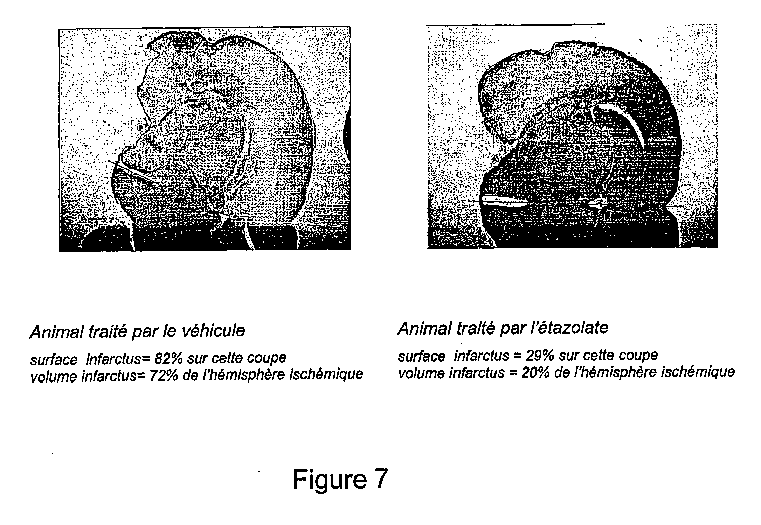

Les résultats obtenus montrent que l'étazolate induit une réduction en moyenne de 28% de la taille de l'infarctus par rapport au contrôle (voir exemple en figure 7). D'autre part, une amélioration de l'hypoactivité a été observée dans le groupe traité à l'étazolate par rapport au groupe contrôle (31% pour le groupe étazolate versus 42% pour le groupe contrôle). De plus l'évaluation neurologique des animaux démontre une amélioration dans le groupe traité par rapport au groupe contrôle.

### Exemple 5 : Test du labyrinthe aquatique (piscine de Morris)

Ce test est utilisé pour évaluer les capacités à mémoriser et à gérer de l'information spatiale chez le rat dans une situation aversive. La tâche consiste pour l'animal à localiser à l'aide des indices distaux une plate-forme « refuge », invisible par immersion dans un bassin rempli d'eau opacifiée. Le dispositif permet d'évaluer la mémoire de référence de l'animal (la plate-forme reste à la même place à chaque jour du test). Ce test permet d'apprécier des performances mnésiques dépendantes des fonctions de l'hippocampe des animaux testés. Notamment, ce test permet de discriminer les performances de rats adultes (10 mois) et celles de rats âgés (30 mois). L'hippocampe est une structure cérébrale dont les fonctions sont altérées précocement dans la maladie d'Alzheimer. Le test de piscine de Morris est donc particulièrement reconnu par l'homme de métier comme permettant d'apprécier les propriétés pharmacologiques de composés destinés à traiter la maladie d'Alzheimer ainsi que les autres pathologies associées à un déficit cognitif. Des rats âgés traités par l'étazolate (3mg/kg et 10mg/kg) administré par voie orale ainsi que des rats traités par le véhicule ont été utilisés pour cette étude. Les performances de ces animaux dans le test de la piscine de Morris ont été comparées à celles d'un groupe contrôle de rats adultes.
Le traitement par 3mg/kg d'étazolate améliore légèrement les performances des rats âgés. Le traitement par 10mg/kg d'étazolate rapproche de façon importante les performances des animaux âgés de celles des animaux adultes.
Ce résultat indique que l'étazolate améliore les propriétés mnésiques et cognitives dépendantes de l'hippocampe, permettant de réduire les déficits de performance liés à l'âge. Ce résultat qualifie l'étazolate pour le traitement des troubles cognitifs liés à l'âge comme la maladie d'Alzheimer notamment.

### Exemple 6 : Utilisation Clinique chez l'Homme

Cet exemple décrit les conditions d'utilisation chez l'homme de l'étazolate pour le traitement de maladies neurodégénératives. Cet exemple illustre le potentiel thérapeutique de l'invention et ses conditions d'utilisation chez l'homme.

Dans cette étude, des doses uniques croissantes d'étazolate (0.5, 1, 2, 5, 10 et 20 mg) ont été administrées par voie orale sous forme de gélules dosées à 0.5 et 5 mg à des groupes différents et séquentiels de huit sujets jeunes et sains, volontaires, de sexe masculin. Cette étude a été réalisée dans un seul centre, en double aveugle et deux des huit sujets ont reçu un placebo. Les paramètres évalués ont été la tolérance clinique (apparition d'effets adverses, de signes cliniques, changement dans la pression artérielle ou la fréquence cardiaque), électrocardiographique (enregistrement de l'ECG) et biologique (hématologie et biochimie sanguine, examen urinaire) pendant les 24h suivant l'administration du produit. Un dosage plasmatique du produit a été réalisé chez chaque sujet à différents temps avant et après l'administration du produit (0,25 - 0,50 - 1,00 - 1,50 - 2,00 - 3,00 - 4,00 - 5,00 - 6,00 - 8,00 -10,00 - 12,00 et 24,00 heures). Un dosage urinaire du produit a également été réalisé à partir des urines collectées avant et après l'administration du produit (4, 4-8, 8-12 et 12-24 heures).

A l'issue de cette phase d'administration de doses croissantes, un groupe supplémentaire de six sujets reçoit à deux reprises une dose d'étazolate : à jeun et au cours d'un repas riche en graisse. L'objectif de cette seconde partie est de comparer l'évolution des taux sanguins du produit entre les deux conditions d'administration. Les paramètres évalués sont la tolérance clinique (apparition d'effets adverses, de signes cliniques, changement dans la pression artérielle ou la fréquence cardiaque), électrocardiographique (enregistrement de l'ECG) et biologique (hématologie et biochimie sanguine, examen urinaire) pendant les 24h suivant l'administration du produit. Un dosage plasmatique du produit est réalisé chez chaque sujet à différents temps avant et après l'administration du produit (0,25 - 0,50 - 1,00 - 1,50 - 2,00 - 3,00 - 4,00 - 5,00 - 6,00 - 8,00 - 10,00 - 12,00 et 24,00 heures). Un dosage urinaire du produit est également réalisé à partir des urines collectées avant et après l'administration du produit (4, 4-8, 8-12 et 12-24 heures).
Une gélule gastro-résistante est également développée pour ce produit de façon à pouvoir l'utiliser dans les études cliniques chez l'homme.

Les résultats obtenus au cours de la première phase d'étude de doses croissantes ont montré que l'étazolate était bien toléré et n'a pas entraîné d'effets secondaires. De plus, les dosages plasmatiques ont confirmé chez l'homme la bonne absorption du produit aux doses fortes.

## Revendications

1. Utilisation de l'étazolate de formule (II) 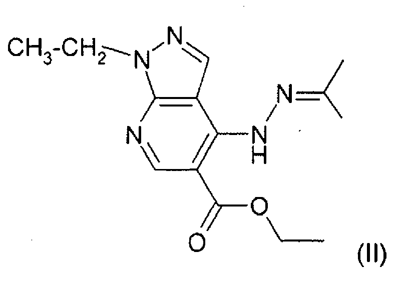 ou d'un sel ou ester de celui-ci, pour la préparation d'une composition pharmaceutique destinée à améliorer la perception cognitive chez un patient humain.

2. Utilisation selon l'une quelconque des revendications précédentes, **caractérisée en ce que** le patient est atteint de la maladie d'Alzheimer, de la maladie de Parkinson ou de la chorée de Huntington.

3. Utilisation selon l'une quelconque des revendications précédentes, **caractérisée en ce que** la composition est administrée par voir orale ou systémique.

4. Composition comprenant l'étazolate de formule (II) 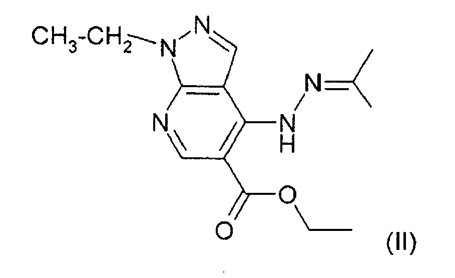 ou un sel ou ester de celui-ci, pour utilisation pour améliorer la perception cognitive chez un patient humain.

## Claims

1. The use of etazolate of formula (II) 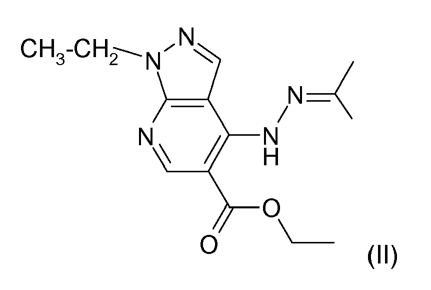 or a salt or ester thereof, for the preparation of a pharmaceutical composition for improving perceptive cognition in a human subject.

2. The use of any one of the preceding claims, **characterized in that** the subject has Alzheimer's disease, Parkinson's disease or Huntington's chorea.

3. The use of any one of the preceding claims, **characterised in that** the composition is administered orally or systemically.

4. A composition comprising etazolate of formula (II) 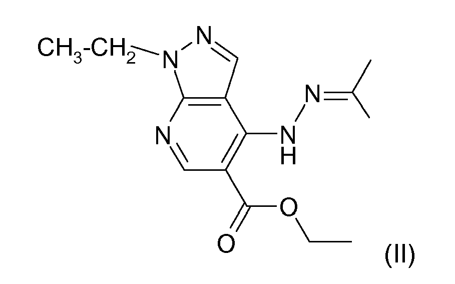 or a salt or ester thereof, for use to improve perceptive cognition in a human subject.

## Patentansprüche

1. Verwendung von Etazolat der Formel (II) 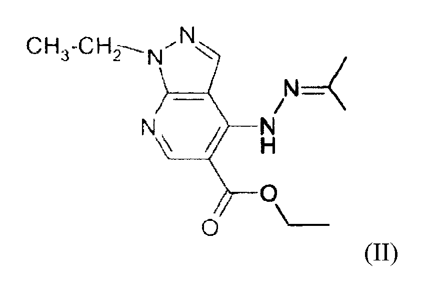 oder einem Salz oder Ester davon für die Zubereitung einer pharmazeutischen Zusammensetzung für eine Verbesserung der kognitiven Wahrnehmung bei einem Humanpatienten.

2. Verwendung nach einem der vorhergehenden Ansprüche, **dadurch gekennzeichnet, dass** der Patient an der Alzheimer Krankheit, Parkinson Krankheit oder Chorea Huntington leidet.

3. Verwendung nach einem der vorhergehenden Ansprüche, **dadurch gekennzeichnet, dass** die Zusammensetzung über einen oralen oder systemischen Weg verabreicht wird.

4. Zusammensetzung, umfassend Etazolat der Formel (II) 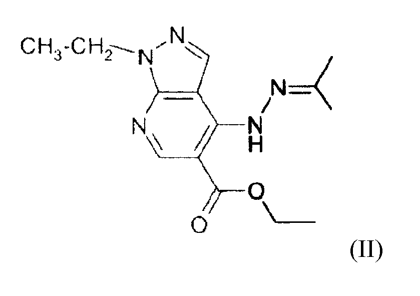 oder ein Salz oder einen Ester davon, zur Verwendung für eine Verbesserung der kognitiven Wahrnehmung bei einem Humanpatienten.
